(19) Europäisches Patentamt
European Patent Office
Office européen des brevets

(11) EP 1 583 781 B1

(12) **FASCICULE DE BREVET EUROPEEN**

(45) Date de publication et mention de la délivrance du brevet:
**02.07.2008 Bulletin 2008/27**

(21) Numéro de dépôt: **03813161.1**

(22) Date de dépôt: **11.12.2003**

(51) Int Cl.:
***C08F 220/12*** *(2006.01)* ***C08F 293/00*** *(2006.01)*
***C08F 2/38*** *(2006.01)* ***C09J 133/06*** *(2006.01)*
***C09D 133/06*** *(2006.01)*

(86) Numéro de dépôt international:
**PCT/FR2003/003669**

(87) Numéro de publication internationale:
**WO 2004/055071 (01.07.2004 Gazette 2004/27)**

(54) **COPOLYMERES A GRADIENT SOLUBLES OU DU MOINS DISPERSIBLES DANS L'EAU COMME DANS LES SOLVANTS ORGANIQUES**

IN WASSER SOWIE ORGANISCHEN LÖSUNGSMITTELN LÖSLICHE ODERZUMINDEST DISPERGIERBARE GRADIENTENCOPOLYMERE

GRADIENT COPOLYMERS SOLUBLE OR AT LEAST DISPERSIBLE IN WATER AS WELL AS IN ORGANIC SOLVENTS

(84) Etats contractants désignés:
**AT BE BG CH CY CZ DE DK EE ES FI FR GB GR HU IE IT LI LU MC NL PT RO SE SI SK TR**

(30) Priorité: **13.12.2002 FR 0215852**

(43) Date de publication de la demande:
**12.10.2005 Bulletin 2005/41**

(73) Titulaire: **ARKEMA FRANCE**
**92700 Colombes (FR)**

(72) Inventeur: **GUERRET, Olivier**
**F-64230 Mazerolles (FR)**

(74) Mandataire: **Bonnel, Claudine et al**
**ARKEMA FRANCE**
**Département Propriété Industrielle**
**420, rue d'Estienne d'Orves**
**92705 Colombes Cedex (FR)**

(56) Documents cités:
**WO-A-99/03894**
**WO-A-02/068487**
**WO-A-02/068550**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 583 781 B1

## Description

L'invention se rapporte au domaine de copolymères amphiphiles, particulièrement au domaine des copolymères amphiphiles à gradient et plus particulièrement au domaine des copolymères à gradients solubles ou dispersibles dans l'eau comme dans les solvants organiques.

Elle concerne aussi un procédé de synthèse de ces copolymères à gradient, un procédé de mise en oeuvre de tels polymères en solution dans l'eau ou dans un mélange eau alcool à des concentration supérieures ou égales à 5%.

L'invention concerne aussi l'utilisation de telles solutions dans des formulations qui peuvent être utilisées dans différents secteurs applicatifs tels que la cosmétique, les peintures aqueuses, l'adhésion sur des surfaces ayant peu d'affinité naturelle pour l'eau, la dispersion de charges minérales.

De manière générale on peut décrire un matériau polymère par les trois grandeurs suivantes : masse moyenne en nombre désignée ci-après par Mn ; masse moyenne en poids désignée ci-après par Mw ainsi que par l'indice de polymolécularité Ip.

Ces trois grandeurs suffisent à décrire un homopolymère ou un copolymère à condition de bien connaître sa composition chimique. En fait, les définitions suivantes vont mettre en évidence qu'un copolymère à gradient doit être décrit bien plus précisément et que les paramètres de sa synthèse seront déterminant pour le décrire correctement.

Dans ce qui suit l'abréviation Tg désigne la température de transition vitreuse d'un polymère.

Dans la présente invention on désignera indifféremment par monomère hydrophile des monomères dont les homopolymères correspondants sont hydrosolubles ou hydrodispersibles ou dont une forme ionique l'est.

Un homopolymère est dit hydrosoluble s'il forme une solution limpide lorsqu'il est en solution à 5% en poids dans l'eau, à 25°C.

Un homopolymère est dit hydrodispersible si, à 5% en poids dans l'eau, à 25°C, il forme une suspension stable de fines particules, généralement sphériques. La taille moyenne des particules constituant ladite dispersion est inférieure à 1 μm et, plus généralement, varie entre 5 et 400 nm, de préférence de 10 à 250 nm. Ces tailles de particules sont mesurées par diffusion de lumière.

Les définitions suivantes sont utiles pour pouvoir distinguer les copolymères à gradients des autres copolymères et polymères :

Polymérisation radicalaire contrôlée :

- Polymérisation radicalaire contrôlée par des Nitroxydes. WO 96/24620 ou WO 00/71501 décrivent les outils de cette polymérisation et leur mise en oeuvre. La compréhension scientifique d'une telle technique de contrôle est décrite par Fischer dans Chemical Reviews, 2001, 101, 3581, par Tordo et Gnanou dans J. Am. Chem. Soc. 2000, 122, 5929 et Hawker dans J. Am. Chem. Soc. 1999, 121, 3904 par exemple.
- Polymérisation par transfert d'atome radicalaire. Décrite dans W0 96 30421, elle procède par l'insertion réversible sur un complexe organométallique dans une liaison de type carbone-halogène.
- Polymérisation radicalaire contrôlée par des dérivés soufrés de type xanthate, dithioesters, trithio carbonates ou carbamates. On pourra se reporter aux documents suivants : FR 01/02848, WO 02/068550, WO 98/01478, WO 99/35177, WO 98/58974, WO 99/31144, WO 97/01478. Une publication de référence dans le domaine étant: Rizzardo & al. Macromolecules, 1998, 31, 5559.

La polymérisation radicalaire contrôlée désigne des polymérisations pour les quelles les réactions secondaires qui conduisent habituellement à la disparition des espèces propageantes (réaction de terminaison ou transfert) sont rendues très peu probables par rapport à la réaction de propagation grâce à un agent de contrôle des radicaux libres. L'imperfection de ce mode de polymérisation réside dans le fait que lorsque les concentrations en radicaux libres deviennent importantes par rapport à la concentration en monomère, les réactions secondaires redeviennent déterminantes et tendent à élargir la distribution des masses.

Gradient

Copolymère à gradient : le copolymère à gradient est un copolymère d'au moins deux monomères obtenu généralement par polymérisation vivante ou pseudo vivante. Grâce à ces modes de polymérisation, les chaînes polymères croissent simultanément et donc incorporent à chaque instant les mêmes ratio de co-monomères. La distribution dans les chaînes polymères des co-monomères dépend donc de l'évolution, pendant la synthèse, des concentrations relatives des co-monomères. On se rapportera aux publications suivante pour une description théorique des copolymères à gradient :T. Pakula & al., Macromol. Theory Simul. 5, 987-1006 (1996) ; A. Aksimetiev & al. J. of Chem. Physics 111,

n°5 ; M. Janco J. Polym. Sci., Part A: Polym. Chem. (2000), 38(15), 2767-2778 ; M. Zaremski, & al Macromolecules (2000), 33(12), 4365-4372 ; K. Matyjaszewski & al. J. Phys. Org. Chem. (2000), 13(12), 775-786 ; Gray Polym. Prepr. (Am. Chem. Soc., Div. Polym. Chem.) (2001), 42(2), 337-338; K. Matyjaszewski Chem. Rev. (Washington, D. C.) (2001), 101 (9), 2921-2990.

Gradient naturel : se dira d'un copolymère à gradient synthétisé en batch à partir d'un mélange initial de co-monomères. La distribution dans la chaîne des divers monomères suit donc une loi déduite de la réactivité relative et des concentrations initiales de monomères. Ces polymères constituent la classe la plus simple de copolymères à gradient car c'est le mélange initial qui définit la propriété finale de produit.

Gradient artificiel : se dira d'un copolymère dont on fera varier la concentration en monomères durant la synthèse par un artifice de procédé.

Gradient de composition : c'est la fonction G définie par

$$\vec{G}(x) = \sum \overrightarrow{[M_i](x)}$$

où x désigne la position normalisée sur la chaîne polymère et $[M_i](x)$ la concentration relative en cette position du monomère $M_i$ (exprimée en mole). Dans le cas de monomères iso réactifs, $[M_i](x)=1/2$.

La fonction G(x) décrit donc la composition du polymère à gradient localement. Deux copolymères peuvent avoir une composition globale équivalente mais des fonctions G(x) très différentes. Les facteurs déterminant les fonctions G(x) sont les coefficients de réactivité relatifs des monomères ($r_i$ pour le monomère $M_i$) (qui dépendent principalement du type de procédé de synthèse (homogène, dispersé) et des solvants...), les concentrations initiales des monomères, les ajouts au cours de la polymérisation de monomères. A titre d'exemple citons le cas d'école d'un copolymère à gradient de styrène ($M_1$)et de l'acide méthacrylique ($M_2$) dans un système de polymérisation homogène. La littérature nous donne r1 = 0,418 et r2 = 0,6.

La variation des concentrations initiales en styrène et en acide méthacrylique permet l'obtention des différents copolymères à gradients ayant donc des chaînes à structures complètement différentes. Ainsi à 10% en acide méthacrylique on obtient un copolymère à gradient très faible dont on ne peut attendre une nanostructuration, à 20% on obtient un copolymère à « tête » hydrophile et queue hydrophobe avec un gradient suffisamment prononcé pour conduire à une nanostructuration. En revanche à 50%, les monomères étant isoréactifs dans ces conditions, le copolymère obtenu est du type alterné.

Malgré le fait que chacun des polymères décrits soit un polymère à gradient de styrène et d'acide méthacrylique, la différence de concentration initiale des monomères conduit à des chaînes de structures complètement différentes conférant aux copolymères des propriétés différentes.

Cet exemple illustre donc l'importance des compositions initiales de monomère sur l'arrangement le long de la chaîne des différents monomères.

Copolymère à blocs :

Il s'agit en fait d'une sous classe de la famille des copolymères à gradient artificiel. $M_i[x]$ est alors le produit d'au moins une fonction Dirac par une fonction monotone ce qui traduit le fait qu'on passe d'un mélange de monomères à un autre dans la chaîne du fait d'un changement subit des monomères dans le milieu réactionnel (stripping du premier mélange ou addition d'au moins un nouveau monomère. Certains copolymères à blocs se différencient par le fait qu'ils possèdent une charnière statistique entre les deux blocs.

Dans WO 02/068550 les auteurs décrivent et revendiquent des polymères à blocs de type (AB)n - coeur (avec n supérieur ou égal à 2) avec éventuellement une charnière entre les blocs A et B, de telles structures correspondent aux courbes GA(x) suivantes :

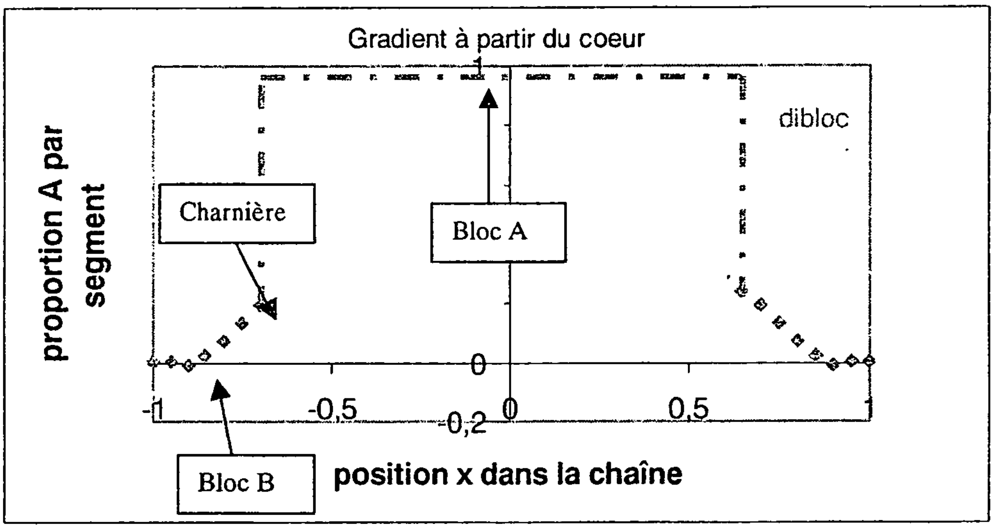


Nous voyons donc que la structure de telle chaîne est :

- symétrique
- la notion de bloc se traduit par une zone au moins de la chaîne ou la composition en A est 1 et en B est 0.
- de telles structures correspondent à un procédé de synthèse basé sur la polymérisation d'un premier mélange de monomère puis d'un second avec éventuellement purification entre les deux étapes du polymère pour éviter le bloc charnière.

De tels composés sont nano-structurés et présentent donc des propriétés physiques et rhéologiques en rapport avec cette nano-structuration :

la température d'ordre désordre est élevée. Jusqu'à cette température, la viscosité du polymère est très élevée et l'une des conséquences est que la mise en solution aqueuse ou en solvant de tels polymères est ardue voire impossible.

Il ressort de ces définitions et de ces exemples que les fonctions G(x), la masse moyenne en nombre et l'indice de polymolécularité sont les trois données nécessaires et suffisantes qui différencient un copolymère à gradient d'un autre. Il ressort aussi que la fonction G(x) est directement déterminée par le procédé de synthèse.

De manière étonnante, la demanderesse a découvert que des polymères à gradient, amphiphiles possédant au moins un monomère hydrophile et au moins un monomère $M_1$ dont l'homopolymère correspondant à une $Tg_1$ inférieure à 20°c présente l'avantage d'être facilement manipulables dans l'eau ou en solvant par rapport à des copolymères à blocs tout en conservant des propriétés rhéologiques intéressantes pour des applications telles que la peinture, les vernis, l'adhésion, la dispersion de charge ou encore les formulations à usage médical, dermatologique ou cosmétique, le monomère $M_1$ pouvant être le monomère hydrophile.

Le premier objet de l'invention est un copolymère à gradients comprenant au moins deux monomères, le premier ($M_1$) dont l'homopolymère correspondant à une $Tg_1$ inférieure à 20°C représente au moins 50% en poids du poids total du copolymère, le second ($M_2$) dont l'homopolymère correspondant à une $Tg_2$ supérieure à 20°c et de préférence supérieure à 50°C représente au plus 50% en poids du poids total du copolymère. De plus au moins un des monomères doit être hydrophile et représenter au moins 5 % en poids du poids total du copolymère.

Le copolymère de l'invention comprend au moins un monomère $M_i$ tel que la probabilité de rencontrer $M_i$ en une position quelconque normalisée x située le long de la chaîne polymère est non nulle.

Préférentiellement, $Tg_1$ est comprise entre -150°C et 20°C et plus préférentiellement comprise entre -120°C et 15°C.

Selon une forme préférentielle de l'invention, le monomère hydrophile représente au moins 10% en poids du poids du copolymère.

En outre, le copolymère à gradient de l'invention présente des masses moyennes comprises entre 5000 g/mol et 1000000 g/mol, des indices de polymolécularité compris entre 1,1 et 2.5, de préférence entre 1,1 et 2.

Les monomères hydrophiles peuvent être choisis parmi les monomères suivants qui sont spontanément hydrophiles ou qu'une simple transformation (quaternarisation d'une amine ou neutralisation d'un acide) rend hydrophile

dans la structure polymère :

- les acides carboxyliques éthyléniques tels que l'acide acrylique, l'acide méthacrylique , l'acide itaconique ou l'acide fumarique.
- les acrylates et méthacrylates de polyéthylène glycol ou de glycol substitués ou non sur leur fonction terminale par des groupements alkyl, phosphates, phosphonates, sulfonate.
- les amides des acides carboxyliques insaturés comme l'acrylamide ou le méthacrylamide et leur dérivés N substitués.
- les acrylates et méthacrylates d'aminoalkyle, les méthacrylamides d'aminoalkyle.
- les anhydrides carboxyliques porteurs d'une liaison vinylique tels que l'anhydride maléique ou l'anhydride fumarique.
- les vinyles amides tels que la vinyle pyrrolidone ou la vinyle acétamide.
- les vinyles amines tels que la vinyle morpholine ou la vinyle amine.
- la vinyle pyridine.

De préférence $M_1$ est choisi parmi les monomères suivants :

- les acrylates d'alkyle en C1-C12 linèaires ou ramifiés,
- les (méth)acrylates de polyéthylène glycol
- les diènes tels que le butadiène ou l'isoprène.

Les autres monomères rentrant dans le copolymère de l'invention sont choisis parmi les monomères suivants :

- les dérivés styréniques
- les dérivés (méth)acryliques conduisant à des polymères de haute Tg tels que l'acrylate de norbornyl ou le méthacrylate de méthyle.
- l'acrylonitrile et le méthacrylonitrile

Les copolymères à gradient de l'invention peuvent être obtenus par la polymérisation radicalaire contrôlée, notamment suivant le mode opératoire décrit ci-après :

1°) On introduit dans un réacteur agité, contenant ou non un solvant, le mélange des monomères à polymériser, un initiateur de polymérisation radicalaire et un agent de contrôle de la polymérisation. Le mélange est mis sous atmosphère de gaz inerte vis-à-vis de la polymérisation radicalaire tel que l'azote ou l'argon. Comme solvant éventuel de polymérisation, on choisira avantageusement les acétates d'alkyle, tels qu'entre autres l'acétate de butyle ou d'éthyle, des solvants aromatiques, des solvants cétoniques ou alcooliques. Dans le cas où le mélange de monomères est miscible à l'eau, celle ci peut être avantageusement utilisée comme solvant.

2°) On porte le mélange sous agitation à la température de polymérisation souhaitée. Cette température est choisie dans une gamme allant de 10°C à 160°C de préférence de 25°C à 130°C. Le choix de la température de polymérisation doit être optimisé en fonction de la composition chimique du mélange de monomères. En effet, certains monomères ayant des constantes cinétiques de propagation très élevée et une affinité pour l'agent de contrôle plus faible doivent être polymérisés à basse température (par exemple dans le cas d'une proportion importante de dérivés méthacryliques, on préférera une polymérisation à température comprise entre 25°C et 80°C.).

3°) Le milieu de polymérisation est éventuellement modifié pendant la polymérisation (avant d'atteindre 90% de conversion des monomères initiaux) par ajout supplémentaire d'un ou plusieurs des monomères du mélange initial. Cet ajout peut être réalisé de différentes manières comprises entre l'addition brutale à l'addition continue sur la durée totale de la polymérisation.

4°) La polymérisation est arrêtée lorsque la conversion souhaitée est atteinte. De cette conversion dépendent la composition globale du polymère et la fonction G(x). De préférence on atteindra plus de 50% de conversion. Atteindre plus de 90% de conversion est encore préféré.

5°) Les éventuels monomères résiduels sont éliminés soit par évaporation soit par ajout d'une quantité d'initiateur classique de polymérisation tel que les dérivés péroxydiques ou azoïques.

Selon l'invention, l'agent de contrôle de la polymérisation est le nitroxyde I. Le procédé selon l'invention consiste en la synthèse des copolymères en présence de nitroxydes I :

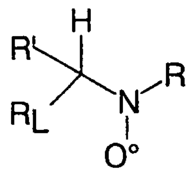

(I)

- où R' et R identiques ou différents, éventuellement reliés de manière à former un cycle sont des groupements alkyl possédant entre 1 et 40 atomes de carbone éventuellement substitués par des groupements hydroxy, alcoxy, amino. En particulier, R et R' seront des groupements ter butyl.
- et où RL est un groupement monovalent de masse molaire supérieur à 16 g/mol, pouvant être un groupement phosphoré ou aromatique, en particulier $R_L$ est un groupement phosphoré et plus particulièrement un groupement phosphonate de formule :

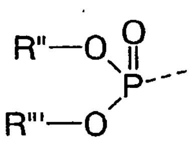

- où R" et R"' identiques ou différents, éventuellement reliés de manière à former un cycle sont des groupements alkyl possédant entre 1 et 40 atomes de carbone éventuellement substitué par des groupements hydroxy, alcoxy, amino. En particulier R" et R"' seront des groupements éthyle.

Les initiateurs de polymérisation conventionnels (azoïque ou peroxyde) peuvent être utilisés en respectant un ratio molaire Nitroxyde/initiateur compris entre 2 et 2,5 si l'on considère qu'une mole de ces initiateurs donne naissance à deux moles de chaînes polymères, ou entre 1 et 1,25 pour des initiateurs monofoctionnels.

Des alcoxyamines de formule générale (II) peuvent être avantageusement choisies pour initier la polymérisation et libérer en même temps le nitroxyde contrôlant cette polymérisation.

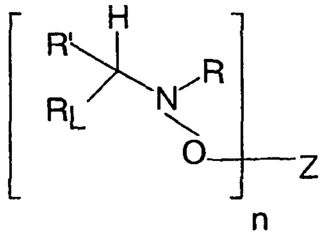

(II)

où n est un entier inférieur ou égal à 8 et de préférence compris entre 1 et 3 et Z est un radical monovalent ou multivalent de type styryle, acryle, ou méthacryle, les autres radicaux ayant les mêmes significations que ci-dessus. La qualité du contrôle de la polymérisation peut être améliorée en additionnant à l'alcoxyamine II du nitroxyde I dans une proportion allant de 0 à 20 % molaires par rapport aux moles de fonctions alcoxyamines (une mole d'alcoxyamine multivalente apporte un nombre de fonctions alcoxyamine proportionnel à sa valence).

Le choix de l'initiateur sera dicté par les besoins de l'application :

- Un initiateur mono-fonctionnel conduira à des chaînes dissymétriques.
- Un initiateur poly-fonctionnel conduira à des macromolécules ayant une symétrie à partir d'un coeur.

Le choix des monomères hydrophiles / hydrophobes sera dicté par l'importance de situer les monomères hydrophiles à endroit précis de la chaîne.

Ainsi, si l'on veut que les motifs hydrophiles soient dans le coeur d'une chaîne polymère, on choisira un initiateur di-fonctionnel et un mélange de monomères tels que la réactivité des monomères hydrophiles est supérieure à celle des hydrophobes, c'est le cas par exemple de l'acide méthacrylique par rapport aux monomères acrylates en général. Dans le cas où l'on veut les motifs hydrophiles à la périphérie, on choisira le cas contraire comme par exemple le couple acrylate/vinyl pyrolidone.

Les copolymères de l'invention sont hydrosolubles ou hydrodispersibles. Ils sont utiles dans les formulations pour peintures, adhésifs, colles et cosmétiques. Ils sont aussi utiles pour la dispersion pigmentaire.

Un des autres objets de l'invention est un procédé de mise en solution aqueuse des copolymères à gradients de l'invention consistant en :

1°) dissoudre le polymère dans une solution de cétone, à un taux de solide compris entre 20 et 90%, de préférence entre 20 et 50%, de préférence on choisira l'acétone ou la méthyléthyl cétone (MEC).

2°) Dans le cas des monomères hydrophiles de type acide, on ajoute à la solution vivement agitée une solution au moins 1 M de base, telle qu'un sel d'ion hydroxonium ($OH^-$), une amine, l'ammoniac, un sel de carbonate ($CO_3^{2-}$) ou d'hydrogénocarbonate ($HCO_3^-$). Dans le cas de monomères hydrophiles de type amine, on ajoute une solution au moins 1 M d'acide tel que l'acide chlorhydrique, bromhydrique, iodhydrique, les acides acétique et propionique, l'acide sulfurique, l'acide phosphorique, l'acide borohydrique. Dans le cas de monomères neutres hydrophiles tels que la diméthyl acrylamide ou la vinyl pyrolidone, la solution obtenue en 1 est laissée inchangée.

3°) On ajoute alors l'eau à la solution obtenue en 1 ou éventuellement en 2 sous vive agitation dans une proportion telle que le taux de solide obtenu soit compris entre 1 et 80%. Eventuellement, l'eau peut être remplacée par des mélanges eau/alcool dans des proportions allant de 99/1 à 50/50.

4°) On évapore la cétone par les techniques classiques d'évaporation, notamment en agitant la solution à 100°C. La concentration se poursuit jusqu'à l'obtention du taux de solide désiré.

En effet, la demanderesse a remarqué que pour réussir la mise en solution aqueuse de tels polymères, il convenait que ceux-ci fussent d'abord dissous dans une solution organique.

Un autre objet de la présente invention concerne la mise en oeuvre de tels polymères en solution dans l'eau ou dans un mélange eau alcool à des concentrations supérieures ou égales à 5%. Les solutions aqueuses ou organiques de tels polymères faisant aussi partie de l'invention.

L'invention concerne aussi l'utilisation de telles solutions dans des formulations qui peuvent être utilisées dans différents secteurs tels que la cosmétique, les peintures, l'adhésion sur des surfaces ayant peu d'affinité naturelle pour l'eau, la dispersion de charges minérales.

Les exemples suivants illustrent l'invention sans en limiter la portée.

<u>**Caractérisation des polymères**</u> **:**

Les masses molaires et leur distribution ont été déterminées par chromatographie d'exclusion stérique (CES), par calibration universelle utilisant des polystyrènes comme standard et les coefficients de Mark-Houwink du PMMA pour les copolymères.

La composition chimique des copolymères peut être déterminée par RMN du proton, spectrométrie UV, infra rouge.

La caractérisation expérimentale du gradient est apportée par la mesure en cours de polymérisation de la composition chimique du polymère.

En effet, pour les polymères préparés par polymérisation vivante ou pseudo vivante, la longueur des chaînes est linéairement liée à la conversion. Autrement dit, si l'on sait quelle proportion d'un monomère $M_1$ s'incorpore à un moment donné de la polymérisation, nous saurons quelle sera la longueur des chaînes à ce moment de la polymérisation et donc nous pouvons connaître la fonction $[M_2](x)$ où x est la position par rapport à la longueur totale de la chaîne.

En prélevant à différents instants de la polymérisation et en faisant la différence de teneur en chaque monomère, on accède ainsi au gradient.

Une autre méthode pour déterminer la fonction gradient est la mesure au fur et à mesure de la conversion des monomères de la Tg du polymère. En effet, en première approximation, la Tg peut être estimée par la relation :

$$\frac{1}{Tg} = \sum \frac{xi}{Tgi}$$

où Tgi désigne la Tg de l'homopolymère du monomère i et xi sa fraction massique.

Ces méthodes reflètent la composition chimique moyenne du matériau obtenu par polymérisation. Il est important de démontrer en outre que toutes les chaînes de polymères ont une composition analogue. Pour cela, on utilisera avantageusement la chromatographie d'absorption liquide qui permet de séparer les chaînes polymères, non plus selon leur poids moléculaire mais selon leur polarité. Cette dernière reflète la composition chimique des polymères constituant le matériau.

**Amorceurs et agent de contrôle utilisés :**

Le radical libre stable utilisé comme agent de contrôle de polymérisation dans les exemples et référencé SG1 correspond à la formule suivante :

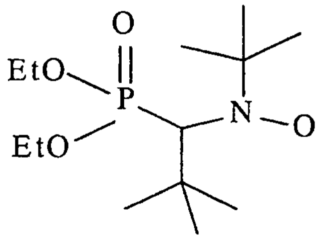

Les alcoxyamines DIAMS et MONAMS cités dans les exemples correspondent aux formules suivantes :

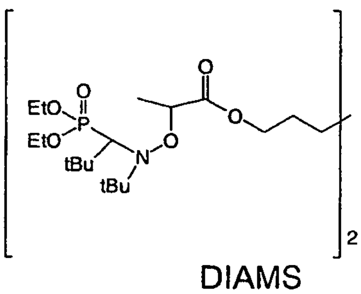

DIAMS

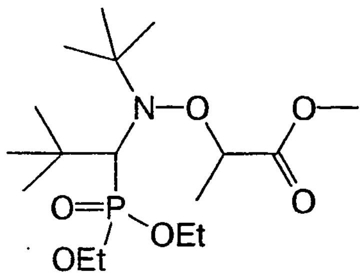

MONAMS

coefficients de réactivité relative de certains monomères utilisés :

Le tableau 1 rappelle certains des coefficients de réactivité relative utilisés dans les exemples :

*Tableau 1 : Coefficients r1,r2 de réactivité des couples de monomères M1/M2*

| | M2 | | | | | | | |
|---|---|---|---|---|---|---|---|---|
| M1 | \ | AE | S | MAM | AMA | AA | Anhydride maléique | NVP |
| | AE | 1 | 0,18 | 0,11 | 0,31 | 0,91 | / | |
| | S | 0,84 | 1 | 0,478 | 0,418 | 0,25 | 0,01 | 17 |
| | MAM | 2,8 | 0,585 | 1 | 1,28 | / | 5,2 | |
| | AMA | 1,25 | 0,6 | 0,48 | 1 | / | / | |
| | AA | 1,31 | 0,136 | / | / | 1 | | |
| | Anhydride maléique | | 0,02 | 0,02 | / | / | / | |
| | NVP | | 0,05 | | | | | |

NVP N vinyl pyrolidone
AE Acrylate d'éthyle
S Styrène
MAM Méthacrylate de méthyle
AA Acide acrylique
AMA Acide méthacrylique

**Exemple 1 : synthèse en masse de copolymère à gradient**

Le mélange de réactifs est le suivant :

- MONAMS : 3,0 g
- SG1 : 0,18 g
- Acrylate d'éthyle (AE) : 480 g soit 80% en poids / poids total de monomères
- Styrène (S) : 60 g soit 10% en poids / poids total de monomères
- Acide méthacrylique (AMA) : 60 g soit 10% en poids / poids total de monomères

L'ensemble des constituants est mélangé, en l'absence de solvant, sous atmosphère d'azote, puis chauffé à une température maintenue entre 110 et 115°C pendant 198 minutes.

Le calcul du gradient par simulation donne la courbe ci-dessous. La validité de ce modèle est donné par le suivi des concentrations relatives des trois monomères par chromatographie gazeuse et analyse RMN des polymères.

On constate qu'à 60% de conversion, la composition chimique du copolymère est 68,4% d'acrylate d'éthyle, 16,1% de styrène et 15,5% d'acide méthacrylique, selon la RMN sur la courbe calculée (69%).

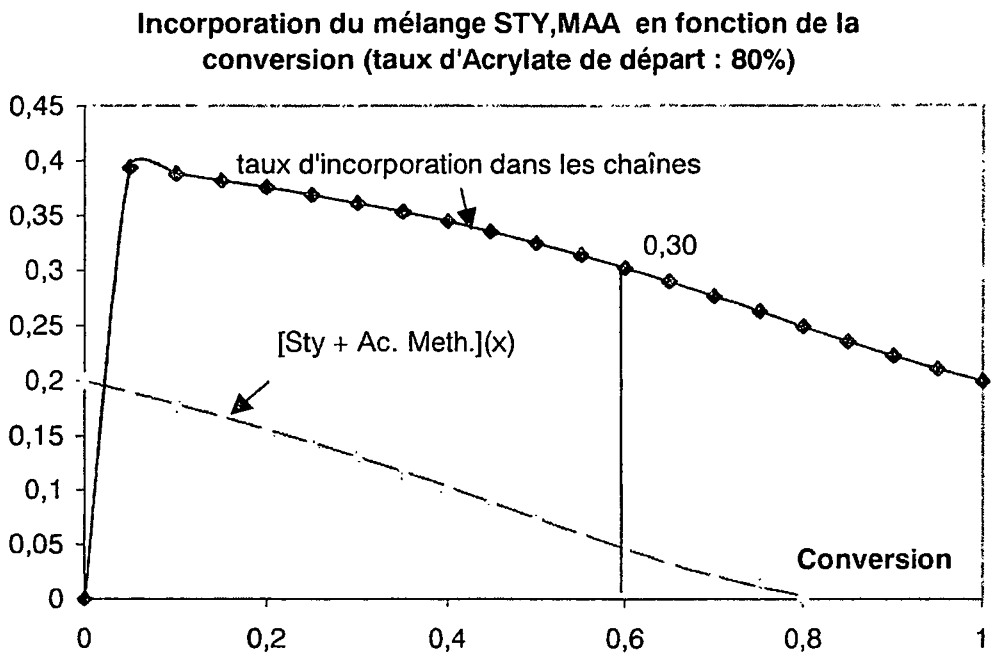


STY et MAA de la figure ci-haut signifient respectivement Styrène et Acide Méthacrylique.

La composition finale du copolymère est la suivante :

- acrylate d'éthyle : 68,4% en poids
- styrène : 16,1 % en poids
- acide méthacrylique : 15,5 % en poids

Par LAC, la trace du polymère montre la faible polydispersité de la composition chimique des chaînes.

La mesure des masses par chromatographie d'exclusion stérique conduit aux résultats suivants : Mn= 32140 g/mol et Mw=51700 g/mol, d'où un indice de polydispersité Ip =1,6.

Une représentation schématique du copolymère obtenu peut être la suivante :

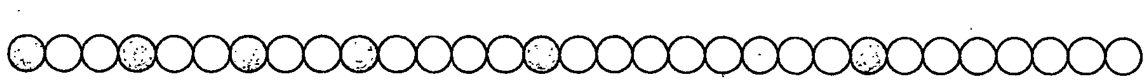

dans laquelle les sphères foncées désignent les enchaînements styrène/acide méthacrylique, et les sphères blanches désignent les enchaînements acrylate d'éthyle.

**Exemple 2 : synthèse en masse de copolymère à gradient**

Selon le mode opératoire décrit à l'exemple 1, on a préparé différents copolymères à partir du mélange de réactifs suivant :
- MONAMS : 3,0 g
- SG1 : 0,18 g
- Styrène : 60 g
- Acide méthacrylique : 60 g
- Acrylate (ou mélange d'acrylate) : 480 g

| Exemple | Acrylate | Caractéristiques du copolymère | Composition finale du (% en poids) |
|---|---|---|---|
| 2a | Acrylate de butyle (Abu) | Mn= 31100 g/mol<br>Mw= 52930 g/mol<br>Ip = 1,7 | Styrène :18<br>Ac. méthacrylique : 22<br>Acrylate de butyle : 60 |
| 2b | Acrylate de méthyle (AMe) | Mn= 32750 g/mol<br>Mw= 61470 g/mol<br>Ip = 1,88 | Styrène :20<br>Ac. méthacrylique : 21<br>Acrylate de méthyle: 59 |
| 2c | Mélange 50/50 en poids acrylate de butyle/acrylate d'éthyle(AE) | Mn= 29690 g/mol<br>Mw= 51630 g/mol<br>Ip = 1,74 | Styrène :18<br>Ac. méthacrylique : 16<br>Acrylates : 66 |

**Exemple 3 : synthèse en présence de solvant**

La même synthèse qu'à l'exemple 1 est réalisée, mais en présence de solvant.
Le mélange de réactifs est le suivant :

- MONAMS : 3,43 g
- SG1 : 0,2 g
- Acrylate d'éthyle : 336 g
- Styrène : 42 g
- Acide méthacrylique : 42 g
- Toluène : 180 g

L'ensemble des constituants est mélangé, dans le toluène comme solvant, sous atmosphère d'azote, puis chauffé à une température maintenue entre 110 et 115°C, pendant 198 minutes.
Le taux de conversion final est de 82%, et le taux de solide obtenu est de 57,2% en poids.
On détermine les résultats analytiques ci-après :
Mn = 30570 g/mol, Mw= 50500 g/mol et Ip = 1,65.
La composition finale du copolymère est donnée par chromatographie d'absorption liquide (LAC), qui indique la similitude de composition avec le copolymère préparé à l'exemple 1 et l'absence d'homopolymère dans les matériaux. Ceci est illustrée par la courbe 1 ci-dessus donnée dans l'exemple 1.

**Exemple 4 : synthèse en présence de solvant**

On réalise, selon le procédé de l'exemple 3, la synthèse d'un nouveau copolymère mais dans un solvant différent : la méthyl éthyl cétone.
La composition initiale du mélange est :

- MONAMS : 4,893 g
- SG1 : 0,2881 g
- Acrylate d'éthyle : 293,8 g

- Acrylate de méthyle : 32, 66 g
- Styrène : 76,8 g
- Acide méthacrylique : 76,8 g
- Méthyléthylcétone : 120 g

Le taux de conversion finale est de 99%, le taux de solide obtenu est de 79,9%.

On détermine les résultats analytiques ci-après :

Mn = 30500 g/mol

Mw= 58000 g/mol

Ip = 1,9

L'incorporation des monomères au cours du temps est mesurée en suivant par chromatographie gazeuse les taux de monomères résiduels au cours du temps :

| temps | | 0 | 75 | 130 | 190 | 290 | 400 |
|---|---|---|---|---|---|---|---|
| Conversion Globale | | 0 | 16 | 30,5 | 49,5 | 85,4 | 99 |
| residuels (%) | AMe | 5,45 | 5,1 | 3,75 | 3,75 | 1,6 | 0,13 |
| | AE | 48,95 | | | | 17,95 | 1,2 |
| | AMA | 12,8 | 12,15 | 4,6 | 2 | 0,35 | 0,08 |
| | S | 12,8 | 12,46 | 6,7 | 3,92 | 0,15 | 0,007 |

* le taux de résiduel total est calculé en tenant compte du solvant quantifié par le taux de solide.

On note que chaque monomère est présent tout au long de la réaction.

La composition finale du copolymère est la suivante :

- acrylate d'éthyle : 34% en poids
- acrylate de méthyle : 34% en poids
- styrène : 16% en poids
- acide méthacrylique : 16% en poids

**<u>Exemple 5</u>**

On prépare une solution aqueuse à 10% de taux de solide, du copolymère préparé à l'exemple 2a.

Pour ce faire, le polymère est préalablement séché à l'étuve. Puis, on dissout 10 g de polymère dans 90 ml d'eau comprenant 1,6 g d'AMP (amino-2-méthyl-2- propanol). On obtient une dispersion aqueuse limpide et très fluide. La taille des particules, mesurée par diffusion (appareil Coulter 4NW) est de 33 nm.

**<u>Exemple 6</u>**

On prépare une solution aqueuse du copolymère préparé à l'exemple 1.

On dissout 10 g de polymère dans 40 g de tétrahydrofurane; on ajoute 1,41 g d'AMP (amino-2-méthyl-2-propanol) dissous dans 10 ml d'eau. La solution s'épaissit. On ajoute alors lentement et sous vive agitation 90 ml d'eau déminéralisée. La solution reste limpide et redevient fluide.

Le solvant est évaporé et on obtient une dispersion aqueuse limpide et fluide. La taille des particules, mesurée par diffusion (appareil Coulter 4NW) est de 199 nm.

**<u>Exemple 7</u> :** exemple d'utilisation d'une solution de copolymère à gradient dans la fabrication de gels en solvant ou aqueux.

10 g du copolymère de l'exemple 2b sont dilués dans 90g de méthyl éthyl cétone. On ajoute au milieu sous agitation 0,8g de propyldiamine et on obtient instantanément un gel.

10 g du copolymère de l'exemple 7 sont dilués dans 90 de méthyl éthyl cétone. On ajoute au milieu 2g d'éthanol diisopropylamine. Le milieu reste fluide. On ajoute 180g d'eau à cette solution. On obtient un gel aqueux.

**Revendications**

1. Procédé d'obtention de copolymère à gradients par la polymérisation radicalaire contrôlée en solution ou en masse, à une température comprise entre 10 et 160°C et de préférence comprise entre 25 et 130°C en présence d'un initiateur de polymérisation radicalaire et d'un agent de contrôle de la polymérisation, d'un mélange de monomères comprenant au moins deux monomères le premier ($M_1$) dont l'homopolymère correspondant à une $Tg_1$ inférieure à 20°C, de préférence comprise entre-150 et 20°C et encore plus préférentiellement comprise entre -120 et 15°C, représentant au moins 50% en poids du poids total du mélange, le second ($M_2$) dont l'homopolymère correspondant à une $Tg_2$ supérieure à 20°C et de préférence supérieure à 50°C représentant au plus 50% en poids du poids total du mélange, au moins un des monomères étant hydrophile et représentant au moins 5 % en poids du poids total du mélange, **caractérisé en que** l'agent de contrôle de la polymérisation est un nitroxyde de formule générale :

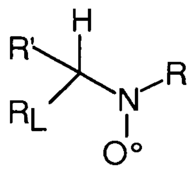

- où R' et R identiques ou différents, éventuellement reliés de manière à former un cycle sont des groupements alkyl possédant entre 1 et 40 atomes de carbone éventuellement substitué par des groupements hydroxy, alcoxy, amino, de préférence R et R' sont des groupements ter butyle
- et où RL est un groupement monovalent de masse molaire supérieur à 16 g/mol, pouvant être un groupement phosphoré ou un groupement aromatique.

2. Procédé suivant la revendication 1, **caractérisé en ce que** l'initiateur de polymérisation et l'agent de contrôle sont remplacés avantageusement par un mélange constitué d'alcoxyamine répondant à la formule générale (II) et de nitroxyde répondant à la formule générale (I) suivantes :

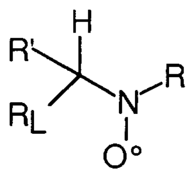
**(I)**

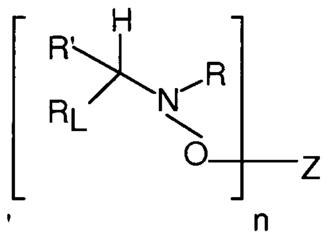
**(II)**

dans lesquelles :

- n est un entier inférieur ou égal à 8 et de préférence compris entre 1 et 3,
- Z est un radical monovalent ou multivalent de porteur de type styryl, acryle, ou méthacryle,
- où R' et R identiques ou différents, éventuellement reliés de manière à former un cycle sont des groupements alkyl possédant entre 1 et 40 atomes de carbone éventuellement substitué par des groupements hydroxy, alcoxy, amino, de préférence R et R' sont des groupements ter butyl
- et où RL est un groupement monovalent de masse molaire supérieur à 16 g/mol pouvant être un groupement

phosphoré ou un groupement aromatique,

le nitroxyde (I) représentant de 0 à 20 % en poids du poids total du mélange.

**3.** Procédé suivant la revendication 1 ou 2, **caractérisé en ce qu'**en particulier RL est un groupement phosphoré et plus particulièrement un groupement phosphonate de formule :

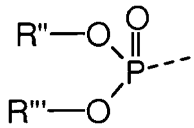

- où R" et R"' identiques ou différents, éventuellement reliés de manière à former un cycle sont des groupements alkyl possédant entre 1 et 40 atomes de carbone éventuellement substitué par des groupements hydroxy, alcoxy, amino, en particulier R" et R"' sont des groupements éthyle,

le nitroxyde (I) représentant de 0 à 20 % en poids du poids total du mélange.

**4.** Procédé selon l'une des revendications précédentes, **caractérisé en ce que** le monomère hydrophile représente au moins 10 % en poids du poids total du mélange.

**5.** Procédé selon l'une des revendications précédentes, **caractérisé en ce que** le monomère hydrophile est choisi dans le groupe comprenant :

- les acides carboxyliques éthyléniques tels que l'acide acrylique, l'acide méthacrylique, l'acide itaconique ou l'acide fumarique,
- les acrylates et méthacrylates de polyéthylène glycol ou de glycol substitués ou non sur leur fonction terminale par des groupements alkyl, phosphates, phosphonates, sulfonate,
- les amides des acides carboxyliques insaturés comme l'acrylamide ou le méthacrylamide et leur dérivés N substitués,
- les acrylates et méthacrylates d'aminoalkyle, les mthacrylamides d'aminoalkyle,
- les anhydrides carboxyliques porteurs d'une liaison vinylique tels que l'anhydride maléique ou l'anhydride fumarique,
- les vinyls amides tels que la vinyl pyrrolidone ou la vinyl acétamide,
- les vinyls amines tels que la vinyl morpholine ou la vinyl amine,
- la vinyl pyridine.

**6.** Procédé selon l'une des revendications précédentes, **caractérisé en ce que** le monomère M1 est choisi parmi les monomères suivants :

- acrylate d'alkyle en C1-C12 linèaires ou ramifiés,
- acrylate ou méthacrylate de polyéthylène glycol,
- les diènes tels que le butadiène ou l'isoprène.

**7.** Copolymère à gradients susceptible d'être obtenu suivant le procédé de l'une des revendications 1 à 6, **caractérisé en ce qu'**il comprend au moins un monomère M tel que la probabilité de rencontrer M en une position quelconque normalisée x située sur la chaîne polymère est non nulle.

**8.** Copolymère selon la revendication 7, **caractérisé en ce qu'**il présente des masses moyennes comprises entre 5000 g/mol et 1000000 g/mol, des indices de polymolécularité compris entre 1,1 et 2,5, de préférence entre 1,1 et 2.

**9.** Procédé de mise en solution aqueuse des copolymères à gradient des revendications 7 ou 8 selon les étapes suivantes :

1°) dissoudre le copolymère dans une solution de cétone telle que l'acétone ou la méthyléthyl cétone (MEC) à un taux de solide compris entre 20 et 90 %, de préférence entre 20 et 50 %,

2°) la solution obtenue en 1 est neutralisée si nécessaire par ajout d'une solution molaire soit d'acide soit de base, le choix acide ou base étant conditionné par la nature chimique du monomère hydrophile,
3°) On ajoute alors l'eau à la solution obtenue en 1 ou éventuellement en 2 sous vive agitation dans une proportion telle que le taux de solide obtenu soit compris entre 1 et 80 %, éventuellement, l'eau peut être remplacée par des mélanges eau/alcool dans des proportions allant de 99/1 à 50/50.
4°) On évapore la cétone jusqu'à l'obtention du taux de solide désiré.

**10.** Solutions aqueuses obtenues suivant le procédé de la revendication 9.

**11.** Utilisation des copolymères à gradients des revendications 7 ou 8 dans les formulations pour peintures, adhésifs, colles ainsi que dans les formulations cosmétiques.

**12.** Utilisation des copolymères à gradients des revendications 7 ou 8 pour la dispersion pigmentaire.

**13.** Utilisation des solutions aqueuses de la revendication 10 dans les formulations pour peintures, adhésifs, colles ainsi que dans les formulations cosmétiques.

**14.** Utilisation des solutions aqueuses de la revendication 10 pour la dispersion pigmentaire.

**Claims**

**1.** Process for producing a gradient copolymer by the solution or bulk controlled radical polymerization, at a temperature of between 10 and 160°C and preferably between 25 and 130°C, in the presence of a radical polymerization initiator and of an agent for controlling the polymerization, of a mixture of monomers comprising at least two monomers, the first ($M_1$), the homopolymer of which corresponding to a $Tg_1$ of less than 20°C, preferably of between -150 and 20°C and more preferably still of between -120 and 15°C, representing at least 50% by weight of the total weight of the mixture, the second ($M_2$), the homopolymer of which corresponding to a $Tg_2$ of greater than 20°C and preferably of greater than 50°C, representing at most 50% by weight of the total weight of the mixture, at least one of the monomers being hydrophilic and representing at least 5% by weight of the total weight of the mixture, **characterized in that** the agent for controlling the polymerization is a nitroxide of general formula:

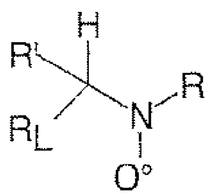

- where R' and R, which are identical or different and which are optionally connected so as to form a ring, are alkyl groups having between 1 and 40 carbon atoms which are optionally substituted by hydroxyl, alkoxy or amino groups; preferably, R and R' are tert-butyl groups;
- and where $R_L$ is a monovalent group with a molar mass of greater than 16 g/mol which can be a phosphorus group or an aromatic group.

**2.** Process according to Claim 1, **characterized in that** the polymerization initiator and the control agent are advantageously replaced by a mixture composed of alkoxyamine corresponding to the following general formula (II) and of nitroxide corresponding to the general formula (I):

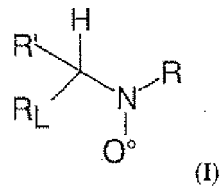

(I)

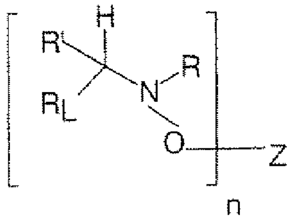

(II)

in which:

- n is an integer of less than or equal to 8 and preferably of between 1 and 3,
- Z is a carrying monovalent or polyvalent radical of styryl, acryloyl or methacryloyl type,
- where R' and R, which are identical or different and which are optionally connected so as to form a ring, are alkyl groups having between 1 and 40 carbon atoms which are optionally substituted by hydroxyl, alkoxy or amino groups; preferably, R and R' are tert-butyl groups;
- and where $R_L$ is a monovalent group with a molar mass of greater than 16 g/mol which can be a phosphorus group or an aromatic group,

the nitroxide (I) representing from 0 to 20% by weight of the total weight of the mixture.

3. Process according to Claim 1 or 2, **characterized in that**, in particular, $R_L$ is a phosphorus group and more particularly a phosphonate group of formula:

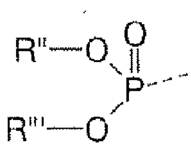

- where R" and R"', which are identical or different and which are optionally connected so as to form a ring, are alkyl groups having between 1 and 40 carbon atoms which are optionally substituted by hydroxyl, alkoxy or amino groups; in particular, R" and R"' are ethyl groups; the nitroxide (I) representing from 0 to 20% by weight of the total weight of the mixture.

4. Process according to one of the preceding claims, **characterized in that** the hydrophilic monomer represents at least 10% by weight of the total weight of the mixture.

5. Process according to one of the preceding claims, **characterized in that** the hydrophilic monomer is chosen from the group consisting of:

- ethylenic carboxylic acids, such as acrylic acid, methacrylic acid, itaconic acid or fumaric acid,
- acrylates and methacrylates of polyethylene glycol or of glycol which are or are not substituted on their end functional group by alkyl, phosphate, phosphonate or sulfonate groups,
- amides of unsaturated carboxylic acids, such as acrylamide or methacrylamide and their N-substituted derivatives,
- aminoalkyl acrylates and methacrylates, and aminoalkylmethacrylamides,
- carboxylic anhydrides carrying a vinyl bond, such as maleic anhydride or fumaric anhydride,
- vinylamides, such as vinylpyrrolidone or vinylacetamide,
- vinylamines, such as vinylmorpholine or vinylamine,
- vinylpyridine.

**6.** Process according to one of the preceding claims, **characterized in that** the monomer $M_1$ is chosen from the following monomers:

- linear or branched $C_1$-$C_{12}$ alkyl acrylates,
- polyethylene glycol acrylate or (meth)acrylate,
- dienes, such as butadiene or isoprene.

**7.** Gradient copolymer capable of being obtained according to the process of one of Claims 1 to 6, **characterized in that** it comprises at least one monomer M such that the probability of encountering M in any standardized position x situated on the polymer chain is nonzero.

**8.** Copolymer according to Claim 7, **characterized in that** it exhibits average masses of between 5000 g/mol and 1 000 000 g/mol and exhibits polydispersity indices of between 1.1 and 2.5, preferably between 1.1 and 2.

**9.** Process for the aqueous dissolution, according to the following stages, of the gradient copolymers of Claims 7 and 8:

1) the copolymer is dissolved in a ketone solution, such as acetone or methyl ethyl ketone (MEK), at a level of solid of between 20 and 90%, preferably between 20 and 50%,
2) the solution obtained in 1 is neutralized, if necessary, by addition of a molar solution either of acid or of base, the acid or base choice being conditioned by the chemical nature of the hydrophilic monomer,
3) water is then added, with vigorous stirring, to the solution obtained in 1 or optionally in 2 in a proportion such that the level of solid obtained is between 1 and 80%; optionally, the water can be replaced by water/alcohol mixtures in proportions ranging from 99/1 to 50/50;
4) the ketone is evaporated until the desired level of solid is obtained.

**10.** Aqueous solutions, obtained according to the process of Claim 9.

**11.** Use of the gradient copolymers of Claims 7 and 8 in formulations for paints, adhesives or glues and in cosmetic formulations.

**12.** Use of the gradient copolymers of Claims 7 and 8 for pigment dispersion.

**13.** Use of the aqueous solutions of Claim 10 in formulations for paints, adhesives or glues and in cosmetic formulations.

**14.** Use of the aqueous solutions of Claim 10 for pigment dispersion.

## Patentansprüche

**1.** Verfahren zum Erhalt von Gradientencopolymer durch radikalische Polymerisation in Lösung oder in Masse bei einer Temperatur im Bereich zwischen 10 und 160 °C und bevorzugt im Bereich zwischen 25 und 130 °C in Gegenwart eines Radikalkettenpolymerisationsinitiators und eines Mittels zur Kontrolle der Polymerisation, einer Mischung aus Monomeren, die mindestens zwei Monomere umfasst, wobei das erste ($M_1$), dessen Homopolymer einer $Tg_1$ kleiner als 20 °C, bevorzugt im Bereich zwischen -150 °C und 20 °C und noch stärker bevorzugt im Bereich zwischen -120 °C und 15 °C entspricht, mindestens 50 Gew.-% des Gesamtgewichts der Mischung darstellt, das zweite ($M_2$), dessen Homopolymer einer $Tg_2$ größer als 20 °C und bevorzugt größer als 50 °C entspricht, höchstens 50 Gew.-% des Gesamtgewichts der Mischung darstellt, wobei mindestens eines der Monomere hydrophil ist und mindestens 5 Gew.-% des Gesamtgewichts der Mischung darstellt, **dadurch gekennzeichnet, dass** das Mittel zur Kontrolle der Polymerisation ein Nitroxid ist, mit der allgemeinen Formel:

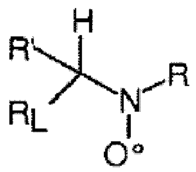

- wobei R' und R, gleich oder verschieden, optional so verbunden, dass sie einen Ring bilden, Alkylgruppen sind, die zwischen 1 und 40 Kohlenstoffatome besitzen, die optional mit Hydroxy-, Alkoxy-, Aminogruppen substituiert sind,

wobei R und R' bevorzugt tert-Butylgruppen sind

- und wobei RL eine monovalente Gruppe mit einer Molekularmasse größer als 16 g/mol ist, die eine Phosphorgruppe oder eine aromatische Gruppe sein kann.

**2.** Verfahren nach Anspruch 1, **dadurch gekennzeichnet, dass** der Polymerisationsinitiator und das Mittel zur Kontrolle vorteilhafterweise durch eine Mischung ersetzt sind, die aus Alkoxyamin besteht, das durch die folgende allgemeine Formel (II) dargestellt wird, und aus Nitroxid, das durch die folgende allgemeine Formel (I) dargestellt wird:

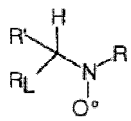

(I)

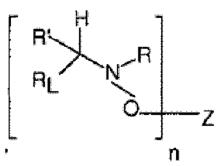

(II)

worin:

- n eine ganze Zahl kleiner oder gleich 8 und bevorzugt im Bereich zwischen 1 und 3 ist,
- Z ein tragender monovalenter oder polyvalenter Rest vom Typ Styryl, Acryl oder Methacryl ist,
- wobei R' und R, gleich oder verschieden, optional so verbunden, dass sie einen Ring bilden, Alkylgruppen sind, die zwischen 1 und 40 Kohlenstoffatome besitzen, die optional mit Hydroxy-, Alkoxy-, Aminogruppen substituiert sind,

wobei R und R' bevorzugt tert-Butylgruppen sind

- und wobei RL eine monovalente Gruppe mit einer Molekularmasse größer als 16 g/mol ist, die eine Phosphorgruppe oder eine aromatische Gruppe sein kann,

wobei das Nitroxid (I) 0 bis 20 Gew.-% des Gesamtgewichts der Mischung darstellt.

**3.** Verfahren nach Anspruch 1 oder 2, **dadurch gekennzeichnet, dass** RL speziell eine Phosphorgruppe und insbesondere eine Phosphonatgruppe ist, mit der Formel:

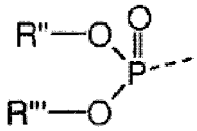

- wobei R" und R"', gleich oder verschieden, optional so verbunden, dass sie einen Ring bilden, Alkylgruppen sind, die zwischen 1 und 40 Kohlenstoffatome besitzen, die optional mit Hydroxy-, Alkoxy-, Aminogruppen substituiert sind, wobei speziell R" und R"' Ethylgruppen sind,

wobei das Nitroxid (I) 0 bis 20 Gew.-% des Gesamtgewichts der Mischung darstellt.

4. Verfahren nach einem der vorhergehenden Ansprüche, **dadurch gekennzeichnet, dass** das hydrophile Monomer mindestens 10 Gew.-% des Gesamtgewichts des Copolymers darstellt.

5. Verfahren nach einem der vorhergehenden Ansprüche, **dadurch gekennzeichnet, dass** das hydrophile Monomer ausgewählt ist aus der Gruppe, umfassend:

- ethylenische Carbonsäuren, wie etwa Acrylsäure, Methacrylsäure, Itaconsäure oder Fumarsäure,
- Polyethylenglykolacrylate- und -methacrylate oder Glycolacrylate und -methacrylate, die auf ihren terminalen Funktionen mit Alkyl-, Phosphat-, Phosphonat-, Sulfonatgruppen substituiert sind oder nicht,
- Amide von ungesättigten Carbonsäuren, wie Acrylamid oder Methacrylamid und deren N-substituierte Derivate,
- Aminoalkylacrylate und -methacrylate, Aminoalkylmethacrylamide,
- Carbonsäureanhydride, die eine Vinylbindung tragen, wie etwa Maleinsäureanhydrid oder Fumarsäureanhydrid,
- Vinylamide, wie etwa Vinylpyrrolidon oder Vinylacetamid,
- Vinylamine, wie etwa Vinylmorpholin oder Vinylamin,
- Vinylpyridin.

6. Verfahren nach einem der vorhergehenden Ansprüche, **dadurch gekennzeichnet, dass** das Monomer $M_1$ aus den folgenden Monomeren ausgewählt ist:

- lineares oder verzweigtes C1-C12-Alkylacrylat,
- Polyethylenglykolacrylat oder -methacrylat,
- Dienen, wie etwa Butadien oder Isopren.

7. Gradientencopolymer, das gemäß dem Verfahren nach einem der Ansprüche 1 bis 6 erhalten werden kann, **dadurch gekennzeichnet, dass** es mindestens ein Monomer M umfasst, bei dem die Möglichkeit, M an irgendeiner standardisierten Position x, die auf der Polymerkette liegt, anzutreffen, nicht null ist.

8. Copolymer nach Anspruch 7, **dadurch gekennzeichnet, dass** es Massenmittel im Bereich zwischen 5.000 g/mol und 1.000.000 g/mol, Polymolekularitätsindizes im Bereich zwischen 1,1 und 2,5, bevorzugt zwischen 1,1 und 2 aufweist.

9. Verfahren zur Löslichmachung in wässriger Lösung der Gradientencopolymere der Ansprüche 7 oder 8 gemäß den folgenden Schritten:

1.) Auflösen des Copolymers in einer Ketonlösung, wie etwa Aceton- oder Methylethylketon (MEK) bei einem Feststoffgehalt im Bereich zwischen 20 und 90 %, bevorzugt zwischen 20 und 50 %,
2.) die in 1 erhaltene Lösung wird gegebenenfalls durch die Zugabe einer molaren Lösung aus entweder einer Säure oder einer Base neutralisiert, wobei die Auswahl der Säure oder Base durch die chemische Natur des hydrophilen Monomers bedingt ist,
3.) dann wird der in 1 oder optional in 2 erhaltenen Lösung Wasser unter starkem Rühren in einem Anteil zugegeben, bei dem der erhaltene Feststoffgehalt im Bereich zwischen 1 und 80 % liegt, optional kann das Wasser durch Wasser/Alkohol-Mischungen in Anteilen ersetzt werden, die von 99/1 bis 50/50 reichen.
4.) Das Keton wird bis zum Erhalt des gesuchten Feststoffgehalts verdampft.

10. Wässrige Lösungen, die gemäß dem Verfahren nach Anspruch 9 erhalten wurden.

11. Verwendung von Gradientencopolymeren nach den Ansprüchen 7 oder 8 in Formulierungen für Farben, Haftmittel, Klebstoffe sowie in kosmetischen Formulierungen.

12. Verwendung der Gradientencopolymere nach den Ansprüchen 7 oder 8 zur Pigmentdispersion.

13. Verwendung der wässrigen Lösungen nach Anspruch 10 in Formulierungen für Farben, Haftmittel, Klebstoffe sowie in kosmetischen Formulierungen.

14. Verwendung der wässrigen Lösungen nach Anspruch 10 zur Pigmentdispersion.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9624620 A
- WO 0071501 A
- WO 9630421 A
- FR 0102848
- WO 02068550 A
- WO 9801478 A
- WO 9935177 A
- WO 9858974 A
- WO 9931144 A
- WO 9701478 A


### Littérature non-brevet citée dans la description


- **FISCHER.** *Chemical Reviews,* 2001, vol. 101, 3581
- **TORDO ; GNANOU.** *J. Am. Chem. Soc.,* 2000, vol. 122, 5929
- **HAWKER.** *J. Am. Chem. Soc.,* 1999, vol. 121, 3904
- **RIZZARDO.** *Macromolecules,* 1998, vol. 31, 5559
- **T. PAKULA.** *Macromol. Theory Simul.,* 1996, vol. 5, 987-1006
- **A. AKSIMETIEV.** *J. of Chem. Physics,* vol. 111 (5
- **M. JANCO.** *J. Polym. Sci., Part A: Polym. Chem.,* 2000, vol. 38 (15), 2767-2778
- **M. ZAREMSKI.** *Macromolecules,* 2000, vol. 33 (12), 4365-4372
- **K. MATYJASZEWSKI.** *J. Phys. Org. Chem.,* 2000, vol. 13 (12), 775-786
- **GRAY.** *Polym. Prepr. (Am. Chem. Soc., Div. Polym. Chem.,* 2001, vol. 42 (2), 337-338
- **K. MATYJASZEWSKI.** *Chem. Rev. (Washington, D. C.,* 2001, vol. 101 (9), 2921-2990